# EUROPEAN PATENT APPLICATION

(11) **EP 2 356 973 A1**
(43) Date of publication of application: **17.08.2011**
(21) Application number: 09831984.1
(22) Date of filing: 08.12.2009
(51) Int. Cl.: A61K 8/26, A61K 8/27, A61Q 1/12, A61Q 19/00

(54) **COSMETIC COMPOSITION**

(30) Priority: 12.12.2008 JP 2008316770
(71) Applicant: KYOWA CHEMICAL INDUSTRY CO., LTD., Kagawa-ken 761-0113 (JP)
(72) Inventor: WANG, Xing Dong, Sakaide-shi Kagawa 762-0012 (JP); SATO, Takatoshi, Sakaide-shi Kagawa 762-0012 (JP)
(74) Representative: Benson, John Everett
(86) International application number: PCT/JP2009/070794
(87) International publication number: WO 2010/067881

(57) **Abstract**

A cosmetic composition having an excellent ultraviolet and infrared protection function, a greatly improved feeling of use and high product stability such as color stability.

The cosmetic composition comprises a polygonal or disk-like ("go" stone-like) aluminum salt hydroxide particle represented by the following formula (1).

Mₐ[Al₁₋ₓ(Zn_{1-y}Fe(III)_{y})ₓ]_{b}A_{c}B_{d}(OH)ₙ·mH₂O (1)

(wherein M is at least one cation selected from Na⁺ and K⁺, A is at least one member selected from organic acid anions having 2 to 10 carbon atoms and containing 1 to 4 carboxyl groups in the molecule, B is at least one inorganic acid anion selected from the group consisting of SO₄²⁻ , NO₃¹⁻ and SiO₃²⁻, and a, b, c, d, m, n, x and y satisfy 0.7 ≦ a ≦ 1.35, 2.7 ≦ b≦ 3.3, 0 ≦ c ≦ 0.5, 1.7 ≦ d ≦ 2.4, 0 ≦ m ≦ 5, 4 ≦ n ≦ 7, 0 < x ≦ 0.66 and 0 < y ≦ 1.0, respectively.)

## Description

### TECHNICAL FIELD

The present invention relates to a cosmetic composition which gives a good feeling of use, has high concealability, is excellent in sunscreen effect and burning sensation reduction effect and facilitates color control. More specifically, it relates to a cosmetic composition which controls color, increases haze, improves spreadability and can provide an ultraviolet absorption effect and an infrared protection effect when it is mixed with a makeup cosmetic product such as foundation or eye-shadow, or a skin-care cosmetic product such as face lotion, skin milk or cream.

### BACKGROUND ART

Makeup cosmetic products assume the aesthetic role of concealing mainly flecks and wrinkles so as to change the color and texture of the skin or part of the face beautiful, and skin-care cosmetic products assume the role of protecting the skin from outside stimuli.

Therefore, when a powder such as a pigment is mixed with a cosmetic, it provides such functions as toning the skin and the hair, concealing flecks and freckles, or protecting the skin from ultraviolet rays. Indian red, yellow iron oxide or chromium oxide is used as a coloring pigment, or titanium oxide or zinc oxide is used as a white pigment. Further, to dilute the coloring pigment, an extender pigment such as talc or mica having high transparency and high adhesion to the skin is used.

In recent years, an ultraviolet protection agent or an infrared reflection agent has often been used to reduce sunburn and burning sensation. Examples of the ultraviolet protection agent include titanium oxide, zinc oxide, iron oxide, cerium oxide, bismuth oxide, zirconium oxide, chromium oxide and tungstic acid. Examples of the infrared reflection agent include zinc oxide, titanium oxide, boron nitride, silver coated talc and silver coated silica.

Further, talc or a spherical silicone powder is often used to improve spreadability at the time of use, that is, application.

However, a cosmetic containing an ultraviolet protection agent or an infrared reflection agent dispersed in an oily ingredient is unsatisfactory in terms of a feeling of use as it is generally sticky, does not spread well and also has a problem with product stability such as solid-liquid separation or caking.

Since the ultraviolet protection agents or the infrared reflection agents enumerated above have low dispersibility and easily agglomerate, they cannot fully exhibit key ultraviolet protection ability or infrared reflection ability in a cosmetic.

Although a coloring material such as a pigment must be mixed with a cosmetic in order to control its color, the amount of the coloring material must be changed for each lot as there are variations in the coloring material by each lot.

Patent Document 1 discloses an aluminum salt hydroxide particle but is utterly silent about the method of preparing a cosmetic composition, the method of providing an infrared reflection effect and the method of improving the spreadability of the cosmetic composition.

Patent Document 2 discloses a cosmetic composition containing a silver solid-solution aluminum salt hydroxide particle at pages 109 to 110 but is utterly silent about the method of providing an ultraviolet and infrared protection effect and the method of improving spreadability.
- (Patent Document 1): WO05/085168
- (Patent Document 2): WO07/004713

### DISCLOSURE OF THE INVENTION

It is an object of the present invention to provide a cosmetic which is excellent in ultraviolet and infrared protection function and feeling of use and has high product stability such as color stability.

The inventors of the present invention have found that a cosmetic which is excellent in feeling of use, product stability and ultraviolet and infrared protection function is obtained by mixing a polygonal or "go" stone-like aluminum salt hydroxide particle (may be simply referred to as "particle" hereinafter) represented by the following formula (1) with a cosmetic. The present invention has been accomplished based on this finding.

The present invention includes the following inventions.
1. A cosmetic composition comprising a polygonal or disk-like ("go" stone-like) aluminum salt hydroxide particle represented by the following formula (1).

   Mₐ[Al₁₋ₓ(Zn_{1-y}Fe(III)_{y})ₓ]_{b}A_{c}B_{d}(OH)ₙ·mH₂O (1)

   (wherein M is at least one cation selected from Na⁺ and K⁺, A is at least one member selected from organic acid anions having 2 to 10 carbon atoms and containing 1 to 4 carboxyl groups in the molecule, B is at least one inorganic acid anion selected from the group consisting of sulfate ion (SO₄²⁻) , nitrate ion (NO₃¹⁻) and silicate ion (SiO₃²⁻), and a, b, c, d, m, n, x and y satisfy 0.7 ≦ a ≦ 1.35, 2.7 ≦ b ≦ 3.3, 0 ≦c≦ 0.5, 1.7 ≦ d ≦ 2.4, 0 ≦ m ≦ 5, 4 ≦ n ≦ 7, 0 < x ≦ 0.66 and 0 <y ≦ 1.0, respectively.)
2. The cosmetic composition in the above paragraph 1, wherein the organic acid anion in the above formula (1) is at least one member selected from the group consisting of oxalate ion, citrate ion, malate ion, tartrate ion, glycerate ion, gallate ion and lactate ion.
3. The cosmetic composition in the above paragraph 1 or 2, wherein the particle is porous or hollow.
4. The cosmetic composition in any one of the above paragraphs 1 to 3, wherein the particle is a composite particle which supports a hydrolysate of at least one compound selected from the group consisting of a zinc compound, an iron compound and a titanium compound on the surface.
5. The cosmetic composition in any one of the above paragraphs 1 to 4, wherein the content of the particle is 0.1 to 30 wt%.
6. The cosmetic composition in any one of the above paragraphs 1 to 5, wherein the particle is baked at 400 to 700°C.
7. The cosmetic composition in any one of the above paragraphs 1 to 6, wherein the particle is obtained by baking a composite particle supporting a hydrolysate of at least one compound selected from the group consisting of a zinc compound, an iron compound and a titanium compound on the surface at 400 to 700°C.

### BRIEF DESCRIPTION OF THE DRAWINGS

Fig. 1 shows a SEM photo of a polygonal aluminum salt hydroxide particle which is particle No. 1 prepared in Synthesis Example 1.
Fig. 2 shows a SEM photo of a "go" stone-like (disk-like) aluminum salt hydroxide particle which is particle No. 6 prepared in Synthesis Example 6.
Fig. 3 shows a SEM photo of a polygonal aluminum salt hydroxide particle which is particle No. 4 prepared in Synthesis Example 4.
Fig. 4 shows a SEM photo of a polygonal aluminum salt hydroxide particle which is particle No. 5 prepared in Synthesis Example 5.
Fig. 5 shows light reflection spectra at ultraviolet to visible and near infrared ranges of powder foundation cosmetic compositions of Example 2;
Fig. 6 is a particle size distribution diagram of the polygonal aluminum salt hydroxide particle which is particle No.1 prepared in Synthesis Example;
Fig. 7 is an X-ray diffraction diagram of the polygonal aluminum salt hydroxide particle which is particle No. 1 prepared in Synthesis Example; and
Fig. 8 is an X-ray diffraction diagram of the "go" stone-like aluminum salt hydroxide particle which is particle No. 6 prepared in Synthesis Example.

### BEST MODE FOR CARRYING OUT THE INVENTION

The present invention will be described in more detail hereinunder.

The aluminum salt hydroxide particle constituting the present invention is represented by the following formula.

Mₐ[Al₁₋ₓ(Zn_{1-y}Fe(III)_{y})ₓ]_{b}A_{c}B_{d}(OH)ₙ·mH₂O (1)

(wherein M is at least one cation selected from Na⁺ and K⁺, A is at least one member selected from organic acid anions having 2 to 10 carbon atoms and containing 1 to 4 carboxyl groups in the molecule, B is at least one inorganic acid anion selected from the group consisting of sulfate ion (SO₄²-), nitrate ion (NO₃¹⁻) and silicate ion (SiO₃²⁻) , and a, b, c, d, m, n, x and y satisfy 0.7 ≦ a ≦ 1.35≦ 2.7 ≦ b≦ 3.3, 0 ≦ c ≦ 0.5, 1.7 ≦ d ≦ 2.4, 0 ≦ m ≦ 5, 4 ≦ n ≦ 7, 0 ≦ x ≦ 0.66 and 0 < y ≦ 1.0, respectively.)

In the formula (1) , M is at least one cation selected from the group consisting of Na⁺ and K⁺ and preferably Na⁺.

In the formula (1), x and y all of which indicate the ratios of Al, Fe (III) and Zn ions satisfy 0 ≦ x ≦ 0.66 and 0 < y ≦ 1.0, respectively. Preferably, they satisfy 0 ≦ x ≦ 0.6 and 0 < y ≦ 1.0, respectively. When x is more than 0.66, Indian red (Fe₂O₃) is formed disadvantageously. The particle of the formula (1) turns from white to flesh color and then from flesh color to red as x and y increase, that is, the content of Fe increases.

In the formula (1) , A is at least one member selected from organic acid anions having 2 to 10 carbon atoms and containing 1 to 4 carboxyl groups in the molecule. A is preferably at least one member selected from the group consisting of oxalate ion, citrate ion, malate ion, tartrate ion, glycerate ion, gallate ion and lactate ion- It is more preferably an oxalate ion or citrate ion, most preferably an oxalate ion.

In the formula (1), B is at least one inorganic acid anion selected from the group consisting of sulfate ion (SO₄²⁻) , nitrate ion (NO₃¹⁻) and silicate ion (SiO₃²⁻) .

According to the knowledge of the inventors of the present invention, the shape of the particle depends on the steric structure of the organic acid anion in the above formula (1). In the present invention, especially when the particle contains Fe(III) and A is an organic acid anion having 2 to 10 carbon atoms and containing 1 to 4 carboxyl groups in the molecule, the particle tends to become polygonal.

The method of synthesizing the disk-like particle is disclosed in Examples 1-B at page 34 of Patent Document 1. To attain the object of the present invention, more preferably, the disk-like particle further supports iron (Fe(III)) or both iron and titanium on the surface.

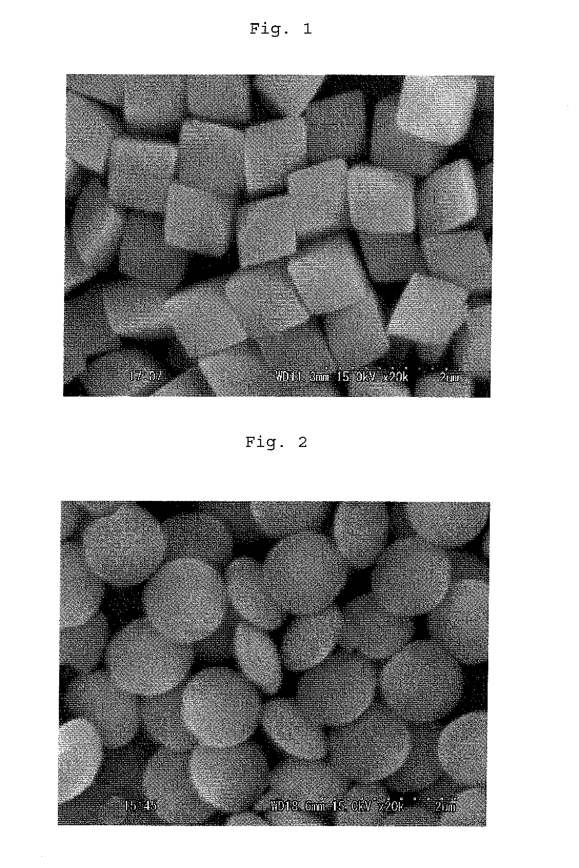

In the cosmetic composition of the present invention, it is preferred to use a polygonal or "go" stone-like (disk-like) particle. Fig. 1 shows a SEM photo of polygonal particles. The particles shown in Fig. 1 are polygonal particles but can be regarded as almost octahedral. Fig. 2 shows a SEM Photo of "go" stone-like particles.

The shape of the particle in the present invention is classified based on its image magnified 10,000 to 20,000 times observed from a SEM photo. One of the measures for specifying the shape of the particle is Wadell' s circularity and sphericity which have been employed in the field of the powder industry.

Wadell's sphericity "s" is defined below, and the particle becomes more spherical as "s" is closer to "1". s = (surface area of a sphere having a volume equivalent to that of particle)/(surface area of particle)

In the present invention, that the shape of the particle is polygonal means that it is similar to an octahedron as shown in Fig. 1, and the above Wadell's sphericity "s" preferably satisfies 0.5 ≦ s ≦ 0: 8.

In the present invention, that the shape of the particle is disk-like ("go" stoke-like) means that it is spheroidal with the short diameter as the axis of rotation as shown in Fig. 2. Stated more specifically, it is preferred that the Wadell's circularity "c" of an projection image of the particle when seen from the direction of the axis of rotation should satisfy 0.95 ≦ c ≦1, and the (short diameter/long diameter) ratio "a" of the ellipse of the section should satisfy 0.05 ≦a ≦0.5.

The average secondary particle diameter of the particles measured by a laser diffraction method is 0.1 to 12 µm, preferably 0.1 to 10µm. It is most preferably 0.2 to 5 µm, particular preferably 0.2 to 2 µm.

As understood from the photos of Fig. 1 and Fig. 2, the polygonal particles of the present invention are uniform in shape and size and have high dispersibility.

It is important that the particles used in the cosmetic composition of the present invention should have a particle size distribution sharpness (D_{R}) of 1 to 1.8 from the viewpoints of improving the dispersibility and developing the satisfactory feeling of use and ultraviolet and infrared protection function of the particles.

The particle size distribution sharpness (D_{R}) is one of the measures for evaluating uniformity in particle diameter and represented by the ratio D₇₅/D₂₅, when D₂₅ represents a particle diameter having a cumulative frequency of 25% from the largest particle diameter of the total number of particles and D₇₅ represents a particle diameter having a cumulative frequency of 75 % while the particle diameter is plotted on the horizontal axis and the cumulative frequency is plotted on the vertical axis.

The range of D_{R} is more preferably 1.01 ≦D_{R} ≦ 1.5, much more preferably 1.02 ≦D_{R} ≦1.4. It is most preferably 1.03 ≦ D_{R} ≦ 1.3.

The specific surface area measured by the BET method of the particle used in the present invention is 0.1 to 300 m²/g, preferably 0.5 to 250 m²/g. The particle used in the present invention is preferably porous or hollow.

### (production of aluminum salt hydroxide particle)

The particle used in the present invention can be produced by adding an alkali hydroxide aqueous solution (such as sodium hydroxide) containing M to a mixed solution of aluminum sulfate, ferric sulfate, zinc oxide, sulfate of M (such as sodium sulfate) in the above formula (1) and an organic acid or organic acid salt (such as oxalic acid) when the inorganic acid ion represented by B in the above formula (1) is a sulfate ion so as to carry out a reaction by heating. If necessary, the obtained particle is separated by filtration, cleaned and dried to obtain a hydrous powder of the particle.

In the above reaction, when the reaction molar ratio [NaOH]/([Fe] + [Al]) is fixed at 3.94, the molar ratio of aluminum to iron ([Al]/[Fe]) preferably satisfies 0.1≦ [Al]/[Fe] ≦2. More preferably, it satisfies 0.1 ≦ [Al]/[Fe] ≦1.6. When [Al] / [Fe] is more than 2, fine iron oxide particles (Indian red) separate out disadvantageously.

When the reaction molar ratio [Al]/[Fe] is fixed at 1.5 in the above reaction, the molar ratio [NaOH]/([Fe] + [Al]) preferably satisfies 3.94 ≦ [NaOH]/([Fe] + [AI]) ≦ 4.4. More preferably, it satisfies 0.1 ≦ [NaOH] / ([Fe] + [Al]) ≦1.6. When [Fe] / [Al] is more than 2, fine iron oxide particles (Indian red) separate out disadvantageously.

When a mixed solution prepared by adding titanium sulfate or titanium chloride and zinc oxide to the above mixed solution is reacted with an alkali hydroxide mixed solution by heating, a solid-solution of an organic acid anion-containing particle which differs from the above particle in composition can be formed. Also, at this point, the reaction molar ratio [Al]/([Fe] + [Zn] + [Ti]) is preferably in the same range as the reaction molar ratio [Al]/[Fe]. [NaOH]/([Fe] + [Al] + [Zn] + [Ti]) is preferably in the same range as [NaOH]/([Fe] + [Al]).

### (composite particle)

When the number of moles of [Fe], [Zn] or [Ti] in the above reaction is made large, a particle (may be referred to as "composite particle" hereinafter) supporting a hydrolysate of a compound in connection with any one of these ions, that is, an oxide, hydroxide, basic salt or acidic salt on the surface of the aluminum salt hydroxide particle can be obtained. In the present invention, particles supporting iron oxide, iron hydroxide (composite hydroxide) , zinc oxide and titanium oxide can be obtained. These composite particles maintain the particle diameters and shapes of particles before they support a hydrolysate.

That is, the aluminum salt hydroxide particle is preferably a composite particle which supports a hydrolysate of at least one compound selected from the group consisting of a zinc compound, an iron compound and a titanium compound on the surface.

The particle supporting a metal hydrolysate on the surface can be obtained by a conventionally known method. That is, it can also be obtained by a method in which various metal compounds are supported on the synthesized aluminum salt hydroxide. For example, it can be obtained by adding an aluminum salt hydroxide particle and a base such as sodium hydroxide to an aqueous solution of titanium sulfate, zinc sulfate or ferric chloride to precipitate a hydrolysate of titanium, zinc or iron on the surface of the aluminum salt hydroxide.

The amount of titanium or zinc supported by the aluminum salt hydroxide is preferably 0.1 to 10 wt% in terms of titanium oxide or zinc oxide. The amount of iron supported by the aluminum salt hydroxide is preferably 0.1 to 3 wt% in terms of iron oxide.

Since the aluminum salt hydroxide supporting a hydrolysate of titanium, zinc or iron on the surface (to be referred to as "composite particle") also has a particle size distribution sharpness D_{R} of 1 to 1.8, its dispersibility in the cosmetic, that is, fat is high. Therefore, as a more excellent ultraviolet and infrared protection function can be obtained and a feeling of use and product stability can be improved by mixing the composite particle with the cosmetic than in a conventional method in which titanium oxide, zinc oxide or iron oxide is used alone, the problem of the present invention is solved advantageously.

Since particle obtained by baking the aluminum salt hydroxide particle or the composite particle in the present invention at 400 to 700°C also exhibit excellent ultraviolet and infrared protection ability and can improve a feeling of use and product stability, it is also useful in solving the problem of the present invention. That is, the aluminum salt hydroxide of the present invention is preferably a particle obtained by baking a composite particle supporting at least one compound selected from the group consisting of a zinc compound, an iron compound and a titanium compound on the surface at 400 to 700°C .

### (surface treatment)

Although the particle in the present invention has excellent dispersibility when it is used directly, the water repellency, oil repellency or dispersibility of the particle can be improved by surface treating it with a perfluoroalkyl phosphoric acid ester salt, silicone such as methyl hydrogen polysiloxane or fluorine compound. By surface treating with an α-amino acid such as lauryl lycine, polysaccharide such as hyaluronic acid or chitosan, protein such as collagen, or glycerophospholipid such as lecithin, the hygroscopic nature, moisturizing action, vital affinity, fluidity, spreadability, oil repellency and dispersibility of the particle can be improved. When it is used in combination with hyaluronic acid, their synergetic effect is large, whereby a greater spreadability improving effect than expected is obtained as compared with when the particle or hyaluronic acid is used alone.

As for the surface treatment of the particle with a surface treating agent, a conventional method known per se may be used as a particle surface treating method. The surface treating agent may be added after any one of thermal reaction (synthesis), separation by filtration, cleaning and drying steps. Further, a surface treating agent may be added when the particle is mixed with a cosmetic. The surface treatment method may be a conventionally known method such as wet method or dry method. The amount of the surface treating agent is 0.01 to 10 parts by weight, preferably 0.05 to 5 parts by weight based on 100 parts by weight of the aluminum salt hydroxide particle.

### (cosmetic composition)

A description is subsequently given of the characteristic feature and production process of the cosmetic composition comprising an aluminum salt hydroxide particle as an effective ingredient of the present invention.

The cosmetic composition of the present invention is useful as a skin-care product, makeup product or fragrance product.

In the cosmetic composition of the present invention, the content of the aluminum salt hydroxide of the formula (1) can be suitably adjusted according to the form of the cosmetic composition such as powder, liquid, cream, cake or stick, or the color of the cosmetic.

For example, when the Fe content of the aluminum salt hydroxide is 20 wt% (y = 1, x = 0.6 in the formula (1)), in the case of skin-care cream, the content of the aluminum salt hydroxide in the cosmetic composition is preferably 0.1 to 5 wt%, more preferably 0.1 to 3 wt%.

In the case of powder foundation, the content of the aluminum salt hydroxide in the cosmetic composition is preferably 0.1 to 65 wt%, more preferably 0.1 to 57 wt%. When the above Fe content is high, the above content can be reduced accordingly.

Further, as the color of an aluminums salt hydroxide supporting Fe on the surface becomes close to red as described above, half of the above content suffices. That is, in the case of skin-care cream, the content of the aluminum salt hydroxide in the cosmetic composition is preferably 0.1 to 30 wt%, more preferably 0.1 to 10 wt%.

Meanwhile, in the case of powder foundation, the content of the aluminum salt hydroxide in the cosmetic composition is preferably 0.1 to 65 wt%, more preferably 0.1 to 25 wt%.

The cosmetic composition of the present invention may be in the form of a powder, liquid, cream, cake or stick.

As components to be mixed together, components which are optionally mixed with an ordinary cosmetic may be used in addition to the above essential ingredients, and these optional components do not reduce the effect of the present invention. The optional components include an oil component, moisturizing agent, emulsifier, inorganic or organic pigment/dye, powder component, amino acids, antiseptic, thickener, pH control agent, fragrance, antiperspirant, antibacterial agent and water. The amounts of these components may be suitably adjusted as long as the object and effect of the present invention are not impaired. Since the pigment/dye may reduce the effect of the present invention as they have light absorption properties at ultraviolet, visible and infrared ranges, attention must be paid to their amounts.

Examples of the above oil component include solid or semisolid oils such as stearic acid, Vaseline and white beeswax, and liquid oils such as olive oil, squalane, silicone oil (dimethicone), liquid paraffin and higher fatty acid esters. These components may be used alone or in combination of two or more.

Examples of the inorganic pigment include Indian red, yellow iron oxide, black iron oxide and titanium oxide. Examples of the powder component include talc, mica, kaolin, cellulose, nylon powders, acrylic powders and silicone powders. Examples of the moisturizing agent include glycerin, glycerin monostearate and sodium hyaluronate. Examples of the emulsifier include cetanol, surfactants, phospholipids and sterol esters. Examples of the antiseptic include methylparaben, benzoic acid, sorbic acid and dehydroacetic acid. Examples of the pH control agent include triethanolamine. They may be used alone or in combination of two or more.

As for the preparation (mixing) methods and the amounts of these components, methods and amounts which are generally used for the oil component, powder component, moisturizing agent, emulsifier, antiseptic and pH control agent may be used.

The use of a pigment/dye component, especially red pigment/dye such as Indian red or iron oxide is preferably minimized only when it is necessary for color control. The amount of the red pigment/dye is more preferably not more than 5 wt%, much more preferably not more than 3 wt%.

The cosmetic composition of the present invention comprising a particle supporting zinc oxide or titanium oxide on the surface as an effective ingredient absorbs a wide ultraviolet range from UVA (320 to 400 nm) to UVC (190 to 290 nm) and rarely causes allergic dermatitis unlike an organic ultraviolet absorbent such as a benzophenone derivative or a dibenzoylmethane derivative.

Since the cosmetic composition of the present invention contains iron as well, it also has an excellent infrared reflection function and therefore can prevent burning sensation due to a rise in the temperature of the skin and smudged makeup caused by perspiration.

Further, the cosmetic composition of the present invention is excellent in spreadability and product stability. That is, skincare cream and foundation comprising the cosmetic composition of the present invention as an effective ingredient spreads well when it is applied to the skin and gives a smooth feel without friction. Even when it is stored for a long time, an oil component, water and a solid component (particle component) do not separate.

The particle used in the present invention changes its color from white to flesh color and then from flesh color to showy pink as amount of Fe increases. To change the color of the cosmetic to flesh color, as the dispersibility of the particle is high, color reproducibility is higher than that of Indian red.

When x is larger than 0 in the above formula (1), a baked product obtained by baking the aluminum salt hydroxide particle at 400°C or higher is excellent in infrared protection effect and emulsifiability. Therefore, a cosmetic comprising this baked product is suitable for solving the problem of the present invention. The baking temperature is preferably 400 to 850°C, more preferably 400 to 700°C.

The present invention includes a method for using the polygonal or disk-like ("go" stone-like) aluminum salt hydroxide particle represented by the formula (1) as an effective ingredient of a cosmetic composition. The present invention also includes a method of improving the ultraviolet protection ability, infrared protection ability, spreadability, feeling of use and stability of a cosmetic composition by using the polygonal or disk-like ("go" stone-like) aluminum salt hydroxide particle represented by the formula (1) as an effective ingredient of the cosmetic composition.

### Examples

The following examples are provided to further illustrate the present invention. The special grade chemicals of Wako Pure Chemical Industries, Ltd. were used unless stated otherwise.

Synthesis Example (preparation of aluminum salt hydroxide particle)

Aluminum salt hydroxide particles (prepared particles Nos. 1 to 8) were prepared by the following method. The characteristic properties of the prepared particles are shown in Table 1. The characteristic properties were measured by the following methods.

### (1) Measurement of particle size distribution, average secondary particle diameter and particle size distribution sharpness D_{R}

Apparatus: Microtrack MT3300 particle size distribution meter (of Leed & Nortrup Instruments Company) Method: laser diffraction scattering method

700 mg of a sample powder was added to 70 ml of an aqueous solution containing 0.2 wt% of sodium hexametaphosphate, dispersed into the solution ultrasonically for 3 minutes and stirred with a stirrer to measure the particle size distribution.

### (2) Observation of particle shape

The shape of each particle was checked from a SEM photo. Apparatus: S-3000N scanning electron microscope (of Hitachi, Ltd.)

Method: acceleration voltage of 15 kV, working distance of 10 mm, magnification of 2,000, 10,000 and 20,000 times

### (3) Measurement of refractive index

Apparatus: 1T Abbe refractometer (of ATAGO)

Method: 5 mg of a sample powder was added to 5 mL of a suitable organic solvent and dispersed into the solvent ultrasonically for 10 minutes, and a transparent portion was applied to the main prism surface to form a thin film so as to obtain its refractive index.

### (4) X-ray diffraction analysis

Apparatus: RINT2200 VX-ray diffraction system (of Rigaku Denki Co., Ltd.)

Method: CU-Kα, angle (2θ) : 5 to 65° , step: 0.02° , scan speed: 4/min, tube voltage: 40 kV, tube current: 20 mV

### (5) Haze measurement

Apparatus: TC-H3DP automatic haze meter (of Tokyo Denshoku Co., Ltd.)

Method: based on JIS-K7136 (ISO14782)

### (prepared particle No. 1)

500 mL of a mixed solution containing 78.5 mL of an aluminum sulfate aqueous solution having a concentration of 1.037 moles/L, 0.1207 mole of ferric sulfate, 0.2 mole of sodium sulfate and 0.04 mole of oxalic acid was stirred at 40°C for 30 minutes.

Then, 244.7 mL of a 3.38 N sodium hydroxide aqueous solution was added to the above mixed solution and stirred at 40°C for 1 hour. Thereafter, a hydrothermal reaction was carried out at 170°C for 3 hours, and the formed particle was separated by filtration, cleaned and dried to obtain a hydrous powder of the particle (prepared particle No. 1).

The reaction molar ratio [Al] / [Fe] was 1.48. The reaction molar ratio [NaOH]/([Fe] + [Al]) was 4.08.

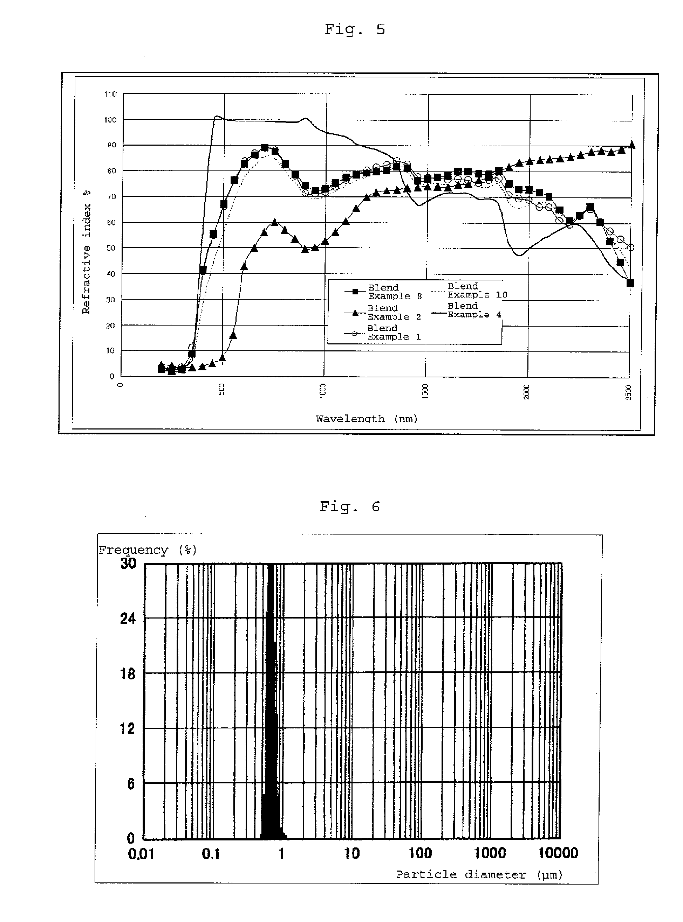

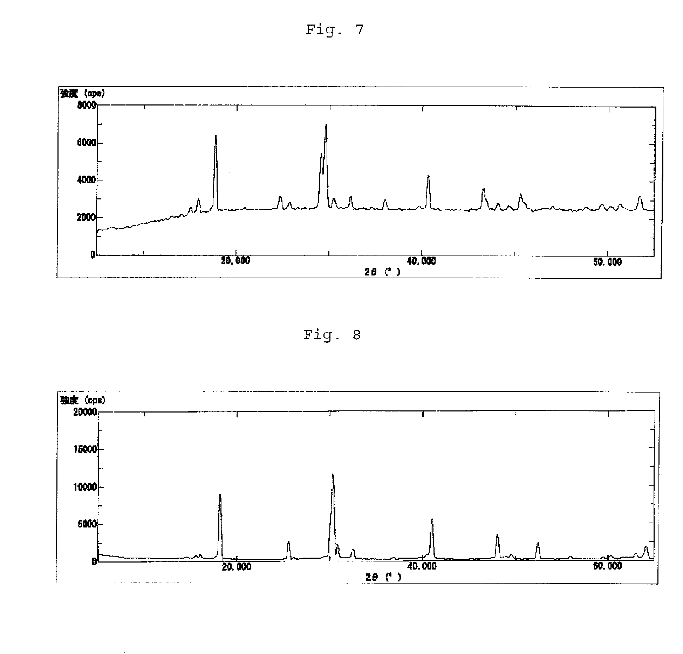

A SEM photo of the prepared particle No.1 is shown in Fig. 1. The particle size distribution of the prepared particle No.1 is shown in Fig. 6, and the X-ray diffraction diagram thereof is shown in Fig. 7.

### (prepared particle No. 2)

500 mL of a mixed solution containing 192.86 mL of an aluminum sulfate aqueous solution having a concentration of 1.037 moles/L, 0.1207 mole of ferric sulfate, 0.2 mole of sodium sulfate, 0.1 mole of sodium metasilicate and 0.04 mole of oxalic acid was stirred at 40°C for 30 minutes.

Then, a 3.38 N sodium hydroxide aqueous solution was added to the above mixed solution until pH became 4 and stirred at 40°C for 1 hour. Thereafter, a hydrothermal reaction was carried out at 180°C for 3 hours, and the formed particle was separated by filtration, cleaned and dried to obtain a hydrous powder of the particle (prepared particle No. 2).

The reaction molar ratio [Al] / [Fe] was 1.48. The reaction molar ratio [NaOH]/([Fe]+[Al]) was 4.08.

### (prepared particle No. 3)

A prepared particle No. 3 was obtained by changing the reaction molar ratio [NaOH]/([Fe]+[Al]) in the synthesis reaction of the above prepared particle No. 1 to 4.40.

### (prepared particle No. 4)

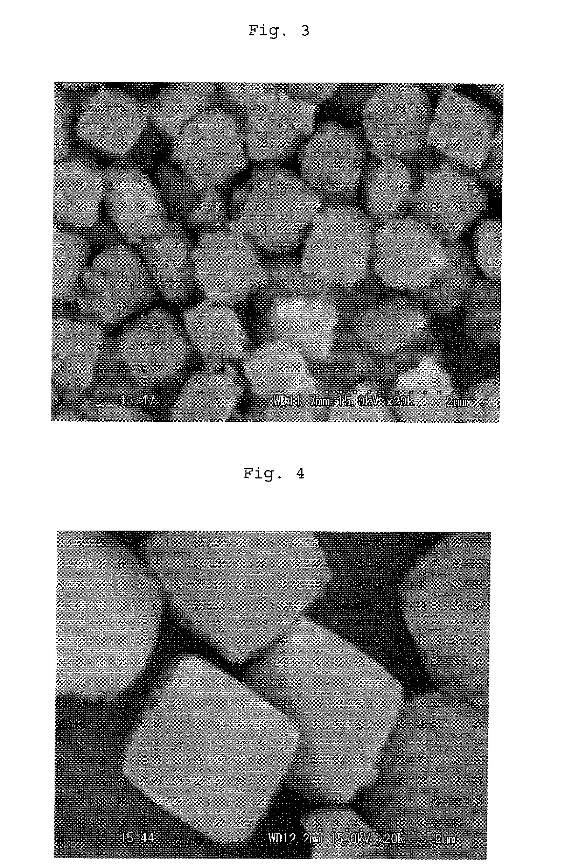

60 g of the prepared particle No. 2 was suspended in 500 ml of ion exchange water, and 60 g of a 30 % titanium sulfate aqueous solution and 120 mL of a 3.37 N sodium hydroxide aqueous solution were added dropwise to the resulting suspension under agitation. Further, a hydrothermal reaction was carried out at 90°C for 2 hours, and the formed particle was separated by filtration, cleaned and dried to obtain a hydrous powder of the particle supporting a hydrolysate of titanium sulfate on the surface (prepared particle No. 4). A SEM photo of the prepared particle No. 4 is shown in Fig. 3.

### (prepared particle No. 5)

500 mL of a mixed solution containing 0.065 mole of an aluminum sulfate aqueous solution, 0.0964 mole of ferric sulfate, 0.04 mole of zinc oxide (first grade, manufactured by Seidou Kagaku Kogyo Co. , Ltd.), 0. 2 mole of sodium sulfate and 0.04 mole of oxalic acid was stirred at 40°C for 30 minutes. Then, 244.7 mL of a 3.38 N sodium hydroxide aqueous solution was added to the above mixed solution and stirred at 40°C for 1 hour. Thereafter, a hydrothermal reaction was carried out at 180°C for 2 hours, and the formed particle was separated by filtration, cleaned and dried to obtain a hydrous powder of the particle (prepared particle No. 5).

The reaction molar ratio [Al] / [Zn] + [Fe] + [Al] was 1.6. The reaction molar ratio [NaOH]/([Zn]+[Fe]+[Al]) was 3.94. A SEM photo of the prepared particle No. 5 is shown in Fig. 4.

### (prepared particle No. 6)

500 mL of a mixed solution containing 192.9 mL (0.2 mole) of an aluminum sulfate aqueous solution having a concentration of 1.037 mole/L, 0.1 mole of sodium sulfate, 0.1 mole of sodium nitrate and 0.04 mole of oxalic acid was stirred at 40°C for 30 minutes.

Then, 244.7 mL of a 3.38 N sodium hydroxide aqueous solution was added to the above mixed solution and stirred at 40°C for 1 hour. Thereafter, a hydrothermal reaction was carried out at 180°C for 3 hours, and the formed particle was separated by filtration, cleaned and dried to obtain a hydrous powder of the particle (prepared particle No. 6). A SEM photo of the prepared particle No. 6 is shown in Fig. 2, and an X-ray diffraction diagram thereof is shown in Fig. 8.

### (prepared particle No. 7)

60 g of the prepared particle No. 6 were suspended in 500 mL of ion exchange water, and 60 g of a 30 % titanium sulfate aqueous solution and 120 mL of a 3.37 N sodium hydroxide aqueous solution were added dropwise to the resulting suspension under agitation. Further, a

hydrothermal reaction was carried out a 90°C for 2 hours, and the formed particle was separated by filtration, cleaned and dried to obtain a hydrous powder of the particle supporting a hydrolysate of titanium sulfate on the surface (prepared particle No. 7).

### (prepared particle No. 8)

60 g of the prepared particle No. 7 was suspended in 500 mL of ion exchange water, 0.02 mole of ferric chloride was added to the suspension, and 25 mL (0.084 mole) of a 3.37 N sodium hydroxide aqueous solution was added dropwise to the suspension under agitation. Further, a hydrothermal reaction was carried out at 90°C for 2 hours, and the formed particle was separated by filtration, cleaned and dried to obtain a hydrous powder of the particle supporting a layer of a hydrolysate of titanium sulfate and a layer of a hydrolysate of ferric chloride on the surface (prepared particle No.8).

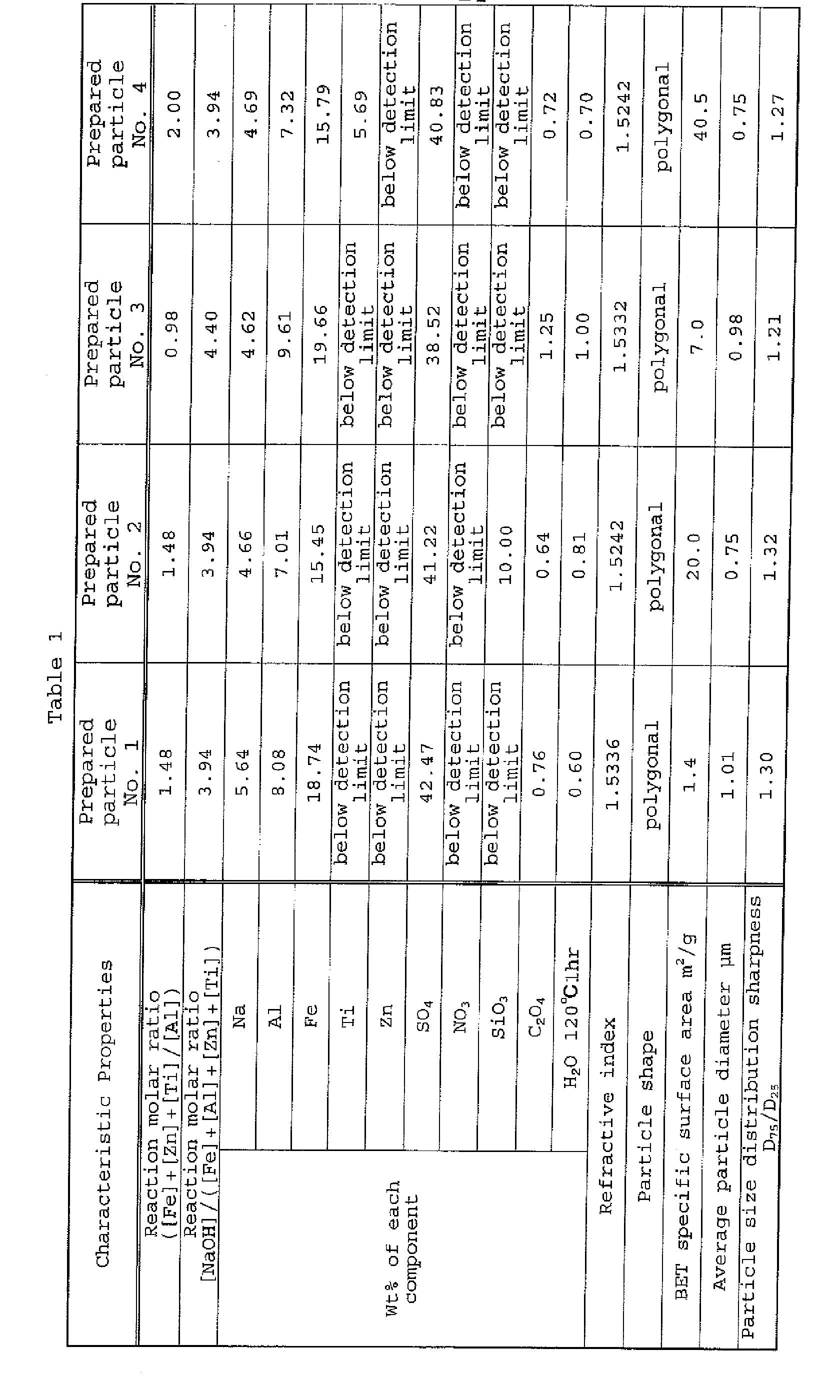

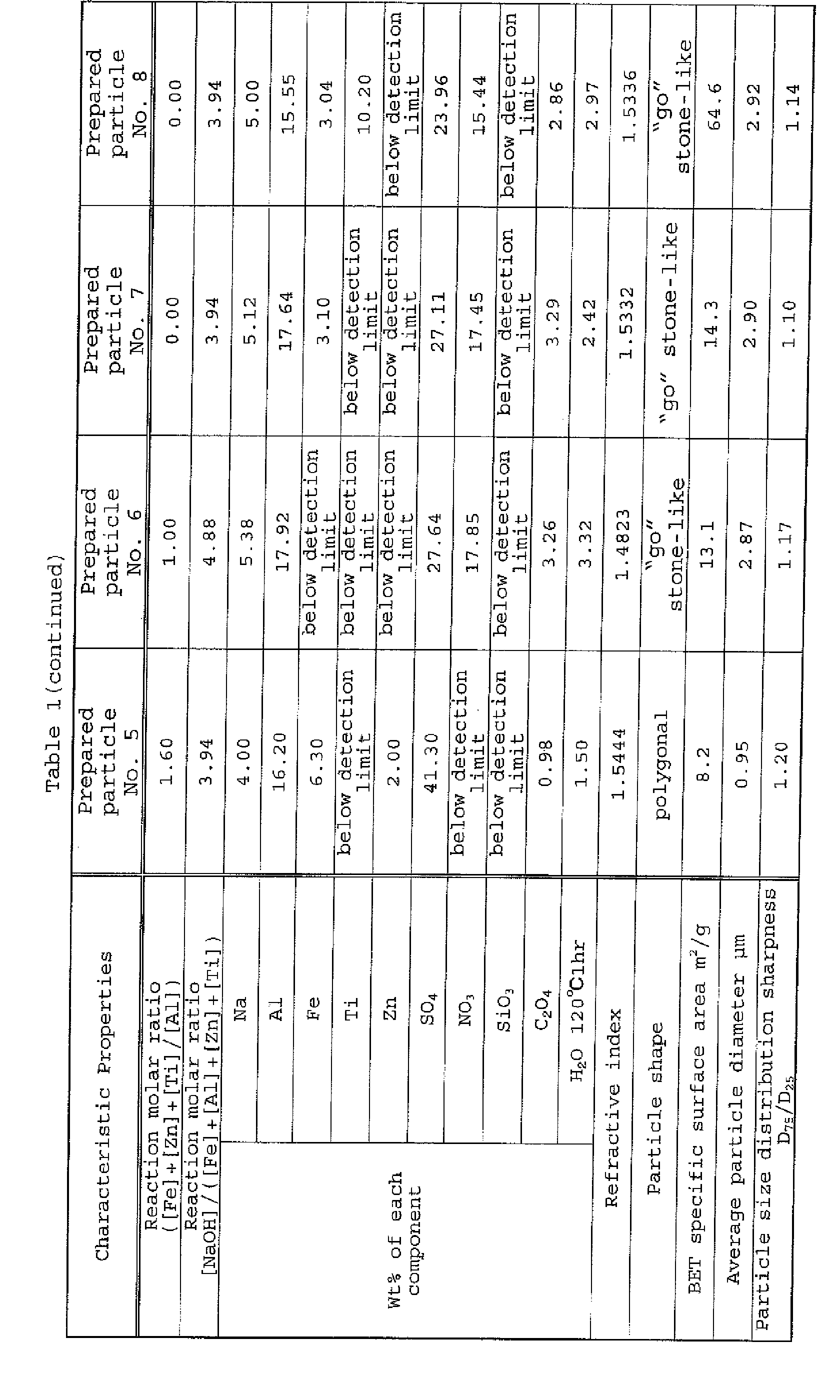

### Example 1 (preparation of skincare cream cosmetic composition)

Cream components shown in Table 2 were prepared, and the prepared particles (prepared particles Nos. 1 to 8) and comparative controls (including conventional commercially available products) were added to these in order to obtain skincare cream cosmetic compositions shown in Table 3.

First-grade chemicals manufactured by Wako Pure Chemical Industries, Ltd. were used except for white beeswax (of Mitsuki Kagaku Co., Ltd.), sodium hyaluronate prepared by a fermentation method (of Senken Co., Ltd.), KSG-210, KF-96A-6cs (of Shin-Etsu Chemical Co., Ltd.) and KF-6017 emulsifier (of Shin-Etsu Chemical Co., Ltd.).

A homo-mixer was used to emulsify the above skincare cream cosmetic compositions, the stirring speed was 10,000 rpm, and the stirring time was 2 hours.

**Table 2**

| Component | Amount % |
|---|---|
| Stearic acid | 2.5 |
| white beeswax | 3.5 |
| Cetanol | 3.5 |
| Squalane | 13.0 |
| Glycerin monostearate | 3.0 |
| Methylparaben | 0.1 |
| Glycerin | 12.0 |
| Triethanolamine | 1.0 |
| Dimeticone KSG-210 | 5.0 |
| Dimethicone KF-96A-6cs | 5.0 |
| Emulsifier KF-6017 | 0.1 |
| Purified water | 51.3 |
| Total | 100.0 |

**Table 3**

| Components % | Blend Example 1 | Blend Example 2 | Blend Example 3 | Blend Example 4 | Blend Example 5 | Blend Example 6 | Blend Example 7 (C. Ex.1) | Blend Example 8 (C.Ex.2) |
|---|---|---|---|---|---|---|---|---|
| Indian red | 0.0 | 0.0 | 0.0 | 0.0 | 0.0 | 0.2 | 0.2 | 0.2 |
| Talc(SG-95) | 0.0 | 0.0 | 3.0 | 0 .0 | 0.0 | 0.0 | 3.0 | 0.0 |
| Hyaluronic acid Na | 0.0 | 0.2 | 0.0 | 0.0 | 0.0 | 0.0 | 0.0 | 0.2 |
| Prepared particles No. 1 | 3.0 | 0.0 | 0.0 | 0.0 | 0.0 | 0.0 | 0.0 | 0.0 |
| Prepared particles No. 2 | 0.0 | 3.0 | 0.0 | 0.0 | 0.0 | 0.0 | 0.0 | 0.0 |
| Prepared particles No. 3 | 0.0 | 0.0 | 3.0 | 0.0 | 0.0 | 0.0 | 0.0 | 0.0 |
| Prepared particles No. 4 | 0.0 | 0.0 | 0.0 | 3.0 | 0.0 | 0.0 | 0.0 | 0.0 |
| Prepared particles No. 6 | 0.0 | 0.0 | 0.0 | 0.0 | 3.0 | 0.0 | 0.0 | 0.0 |
| Prepared particles No. 8 | 0.0 | 0.0 | 0.0 | 0.0 | 0.0 | 3.0 | 0.0 | 0.0 |
| Titanium oxide TTO-F-6 | 0.0 | 0.0 | 0.0 | 0.0 | 0.0 | 3.0 | 3.0 | 3.0 |
| Cream components | 97.0 | 96.8 | 94.0 | 97.0 | 97.0 | 93.8 | 93.8 | 96.6 |
| Total | 100.0 | 100.0 | 100.0 | 100.0 | 100.0 | 100.0 | 100.0 | 100.0 |

| | | | | | | | | |
|---|---|---|---|---|---|---|---|---|
| C.Ex.: Comparative Example | | | | | | | | |

The following evaluations were made on the prepared skincare cream cosmetic compositions, and the results are shown in Table 4.

### (1) Evaluation of concealability

An applicator (YBA-4 of Yoshimitsu Seiki Co., Ltd.) was used to apply each skincare cream cosmetic composition to a glass sheet having a thickness "t" of 1.0 mm to a thickness of 50 µm so as to prepare a test piece. The total light transmittance and diffusion permeability of the test piece were measured by using an automatic haze meter (TC-H3DP of Tokyo Denshoku Co. , Ltd. ) to calculate its haze in accordance with JIS-K7136 (ISO14782) The concealability was evaluated based on this haze value.

### (2) Feeling of use

Spreadability and application ease at the time of using each cosmetic composition were evaluated by 10 panelists. They were evaluated based on five criteria from 1 as "bad" to 5 as "good", and the average number of points of the 10 panelists was taken as the number of evaluation points. Therefore, as the number of evaluation points increases, a feeling of use (spreadability and application ease) becomes better.

### (3) Color stability

The prepared particles and comparative controls (including conventional commercially available products) were added to cream components and stirred by means of a homo-mixer at a stirring speed of 10,000 rpm for 60 minutes to be emulsified, and Y, x and y of the resulting products were measured with a colorimetric color difference meter (ZE-2000 of Nippon Denshoku Industries Co., Ltd.). The addition step and the emulsification step were carried out 3 times for each of the prepared particles so as to obtain the average value of the measurement data and standard deviation, and color stability was evaluated based on the variation coefficient (=standard deviation/average value). As a result, as the variation coefficient becomes smaller, color reproducibility after emulsification becomes higher, which means higher color stability.

### (4) Emulsion stability

After each of the prepared skincare cream cosmetic compositions was put into a plastic case to be left at normal temperature for 2 months, its emulsion state was checked visually. The emulsion state was evaluated based on the following three criteria.
○: emulsion state is maintained
Δ : emulsified at first glance but when it is applied to the skin, water separates
×: it is seen that water and an oil component apparently separate from each other in case

In the above evaluations, as comparative controls to the prepared particles Nos. 1 to 8, Indian red (Fe₂O₃) and fine particulate titanium oxide (average primary particle diameter of 15 nm, trade name: MT-150W of Teika Co., Ltd.) were used in Blend Examples 7 and 8.

**Table 4**

| Characteristic properties | | Blend Example 1 | Blend Example 2 | Blend Example 3 | Blend Example 4 | Blend Example 5 | Blend Example 6 | Blend Example 7 (C. Ex. 1) | Blend Example 8 (C.Ex.2) |
|---|---|---|---|---|---|---|---|---|---|
| Total light transmittance % | | 80.0 | 78.5 | 84.1 | 84.0 | 82.4 | 97.3 | 79.6 | 82.1 |
| Diffusion permeability % | | 66.8 | 62.3 | 61.0 | 59.7 | 58.5 | 72.6 | 55.8 | 36.4 |
| Haze % | | 83.5 | 79.4 | 72.5 | 71.1 | 71.0 | 74.6 | 70.1 | 44.3 |
| Color average value (n=5) | < Y > | 58.76 | 58.59 | 58.15 | 58.24 | 79.86 | 78.88 | 57.53 | 79.71 |
| | < X > | 0.3671 | 0.3703 | 0.3704 | 0.3674 | 0.3167 | 0.3124 | 0.4112 | 0.3176 |
| | < y > | 0.3712 | 0.3741 | 0.3740 | 0.3677 | 0.3258 | 0.3248 | 0.3156 | 0.3269 |
| Color reproducibil ity (n=5) | σ_{γ} / < Y > % | 5.28 | 5.33 | 4.40 | 6.78 | 4.31 | 6.20 | 19.40 | 9.55 |
| | σ_{×} /< x > % | 1.59 | 1.87 | 1.88 | 2.14 | 1.59 | 2.68 | 3.83 | 3.25 |
| | σ_{y} / y > % | 0.12 | 0.09 | 0.16 | 0.11 | 0.08 | 0.16 | 0.59 | 0.21 |
| Feeling of use (spreadability, application ease) | | 4.0 | 3.9 | 3.4 | 4.2 | 3.6 | 3.2 | 3.2 | 2.9 |
| Emulsion stability | | O | ○ | ○ | ○ | ○ | Δ | Δ | X |

| | | | | | | | | | |
|---|---|---|---|---|---|---|---|---|---|
| C.Ex.: Comparative Example | | | | | | | | | |

It is understood from Table 4 that the skincare cream cosmetic compositions (Blend Examples 1 to 6) of the present invention have high concealability due to great haze and high spreadability at the time of use as compared with conventional products (Blend Examples 7 and 8).

The color reproducibility of the skincare cream cosmetic compositions of the present invention is higher than that of conventional products in terms of Y, x and y. That is, the color of the skincare cream cosmetic composition is stable under certain emulsification and stirring conditions. Therefore, color control at the time of production is easy. Further, even when it is left at normal temperature for 2 months, its emulsion state is stable without the separation of water and an oil component.

### Example 2 (preparation of powder foundation cosmetic composition)

The particles obtained in Preparation Examples (prepared particles Nos. 1 to 5 and 8) , a particle obtained by baking the prepared particle No. 4 at 700°C and grinding it (prepared particle No. 9) and comparative controls (including conventional commercially available products) were put into a Henschel mixer to be mixed together, and powder foundation basic components shown in Table 5 which were pre-mixed together uniformly at 70 to 80°C were gradually added to these powders. After addition, they were further mixed together for 5 minutes to obtain powder foundation cosmetic compositions shown in Table 6.

First grade chemicals of Wako Pure Chemical Industries, Ltd. were used except for Matsumoto Microsphere (of Matsumoto Yushi Seiyaku Co., Ltd.) and TTO-F-6 titanium oxide (of Ishihara Sangyo Co., Ltd.).

**Table 5**

| Component | Content wt% |
|---|---|
| Matsumoto Microsphere M-100 | 47 |
| Silicone treated talc | 23 |
| Silicone treated mica | 21 |
| Squalane | 9 |
| Total | 100 |

**Table 6**

| Component | Blend Example 8 | Blend Example 9 | Blend Example 10 | Blend Example 11 | Blend Example 12 | Blend Example 13 | Blend Example 14 (C.Ex.3) | Blend Example 15 (C.Ex. 4) |
|---|---|---|---|---|---|---|---|---|
| Indian red | 0 | 0 | 0 | 3 | 3 | 0 | 0 | 3 |
| Prepared particles No. 1 | 57 | 0 | 0 | 0 | 0 | 0 | 0 | 0 |
| Prepared particles No. 2 | 0 | 57 | 0 | 0 | 0 | 0 | 0 | 0 |
| Prepared particles No. 4 | 0 | 0 | 57 | 0 | 0 | 0 | 0 | 0 |
| Prepared particles No. 5 | 0 | 0 | 0 | 54 | 0 | 0 | 0 | 0 |
| Prepared particles No. 8 | 0 | 0 | 0 | 0 | 54 | 0 | 0 | 0 |
| Prepared particles No. 9 | 0 | 0 | 0 | 0 | 0 | 57 | 0 | 0 |
| TTO-F-6 titanium oxide | 0 | 0 | 0 | 0 | 0 | 0 | 57 | 54 |
| Powder foundation basic components | 43 | 43 | 43 | 43 | 43 | 43 | 43 | 43 |
| Total | 100 | 100 | 100 | 100 | 100 | 100 | 100 | 100 |

| | | | | | | | | |
|---|---|---|---|---|---|---|---|---|
| C.Ex.: Comparative Example | | | | | | | | |

Table 7 shows the evaluation results of the prepared powder foundation cosmetic compositions which were made in the same manner as in Example 1. The ultraviolet and infrared protection function of each of the compositions was evaluated by measuring reflection spectra at 200 to 2, 500 nm of a pellet having a thickness of 1 mm obtained by press molding with a spectrophotometer (150-20 of Hitachi, Ltd.). The obtained reflection spectra are shown in Fig. 5.

**Table 7**

| Characteristic properties | | Blend Example 8 | Blend Example 9 | Blend Example 10 | Blend Example 11 | Blend Example 12 | Blend Example 13 | Blend Example 14 (C.Ex.3) | Blend Example 15 (C.Ex.4) |
|---|---|---|---|---|---|---|---|---|---|
| Total light transmittance % | | 3.6 | 3.2 | 3.5 | 6.2 | 31.4 | 4.1 | 7.6 | 2.6 |
| Diffusion permeability % | | 3.3 | 2.9 | 3.2 | 5.6 | 29.1 | 3.8 | 6.7 | 2.2 |
| Haze % | | 91.7 | 90.6 | 91.4 | 90.3 | 92.7 | 92.7 | 88.2 | 84.6 |
| Color average value (n=5) | < Y > | 52.15 | 52.33 | 53.01 | 51.77 | 16.34 | 53.25 | 78.11 | 16.54 |
| | < X > | 0.3789 | 0.3796 | 0.3802 | 0.3842 | 0.4112 | 0.3822 | 0.3081 | 0.4040 |
| | < y > | 0.3754 | 0.3787 | 0.3766 | 0.3765 | 0.3292 | 0.3793 | 0.3345 | 0.3228 |
| Color reproducibility (n=5) | σ_{γ} / < Y > % | 5.99 | 6.52 | 5.23 | 7.86 | 3.10 | 3.52 | 12.34 | 18.12 |
| | σₓ /<x> % | 1.66 | 2.01 | 1.64 | 1.96 | 1.26 | 1.14 | 3.65 | 4.21 |
| | σ_{y} /< y >% | 0.31 | 0.16 | 0.22 | 0.12 | 0.08 | 0.06 | 1.02 | 0.64 |
| Feeling of use (spreadability, application ease) | | 3.8 | 4.2 | 4.1 | 3.5 | 4.0 | 4.2 | 3.1 | 2.7 |

| | | | | | | | | | |
|---|---|---|---|---|---|---|---|---|---|
| C.Ex.: Comparative Example | | | | | | | | | |

It is understood from Table 7 that the powder foundation cosmetic compositions (Blend Examples 8 to 13) of the present invention have high concealability due to great haze and high spreadability at the time of use as compared with Blend Examples 14 and 15 of the prior art. The color reproducibility of the powder foundation cosmetic compositions of the present invention is higher than that of conventional products in terms of Y, x and y. That is, the color is stable under certain mixing conditions. Therefore, color control at the time of production is easy. It is understood from the reflection spectra of Fig. 5 that the powder foundation cosmetic composition of the present invention has both an ultraviolet absorption effect and an infrared protection effect.

### Effect of the Invention

The cosmetic composition of the present invention is excellent in ultraviolet and infrared protection function. The cosmetic composition of the present invention is also excellent in spreadability and feeling of use. The cosmetic composition of the present invention has high product stability such as color stability and emulsion stability.

## Claims

1. A cosmetic composition comprising a polygonal or disk-like ("go" stone-like) aluminum salt hydroxide particle represented by the following formula (1).
Mₐ[Al₁₋ₓ(Zn_{1-y}Fe(III)_{y})ₓ]_{b}A_{c}B_{d}(OH)ₙ·mH₂O (1)
(wherein M is at least one cation selected from Na⁺ and K⁺, A is at least one member selected from organic acid anions having 2 to 10 carbon atoms and containing 1 to 4 carboxyl groups in the molecule, B is at least one inorganic acid anion selected from the group consisting of sulfate ion (SO₄²⁻), nitrate ion (NO₃¹⁻) and silicate ion (SiO₃²⁻) , and a, b, c, d, m, n, x and y satisfy 0.7 ≦ a ≦ 1.35, 2.7 ≦ b ≦ 3.3, 0 ≦ c ≦ 0.5, 1.7 ≦d ≦2.4, 0 ≦ m ≦ 5, 4 ≦ n ≦ 7, 0 < x≦ 0.66 and 0 < y ≦ 1.0, respectively.)

2. The cosmetic composition according to claim 1, wherein the organic acid anion in the above formula (1) is at least one member selected from the group consisting of oxalate ion, citrate ion, malate ion, tartrate ion, glycerate ion, gallate ion and lactate ion.

3. The cosmetic composition according to claim 1 or 2, wherein the aluminum salt hydroxide particle is porous or hollow.

4. The cosmetic composition according to any one of claims 1 to 3, wherein the aluminum salt hydroxide particle is a composite particle which supports a hydrolysate of at least one compound selected from the group consisting of a zinc compound, an iron compound and a titanium compound on the surface.

5. The cosmetic composition according to any one of claims 1 to 4, wherein the content of the aluminum salt hydroxide particle is 0.1 to 30 wt%.

6. The cosmetic composition according to any one of claims 1 to 5, wherein the aluminum salt hydroxide particle is a particle baked at 400 to 700°C.

7. The cosmetic composition according to any one of claims 1 to 6, wherein the aluminum salt hydroxide particle is obtained by baking a composite particle supporting a hydrolysate of at least one compound selected from the group consisting of a zinc compound, an iron compound and a titanium compound on the surface at 400 to 700°C.
